# EUROPEAN PATENT APPLICATION

(11) **EP 4 748 330 A1**
(43) Date of publication of application: **27.05.2026**
(21) Application number: 25207386.1
(22) Date of filing: 08.10.2025
(51) Int. Cl.: A61B 18/14, A61N 1/06

(54) **TREATMENT TIP FOR SCALP CARE HANDPIECE AND HANDPIECE INCLUDING THE SAME**

(30) Priority: 25.11.2024 KR 20240169347
(71) Applicant: VIOL Co. Ltd., Bundang-gu Seongnam-si, Gyeonggi-do, 13510 (KR)
(72) Inventor: LEE, Sangjin, 13510 Seongnam-si, Gyeonggi-do (KR); PARK, Jeongyeol, 13510 Seongnam-si, Gyeonggi-do (KR)
(74) Representative: Stöckeler, Ferdinand

(57) **Abstract**

Disclosed are a treatment tip for a scalp care handpiece and a handpiece including the same. The treatment tip includes a cap part having a tip surface formed with a plurality of needle entry/exit holes, a cooling plate part mounted on the cap part, and a pair of terminal parts located on both sides of the cap part and accommodating both ends of the cooling plate part. The treatment tip provides a function capable of effectively cooling the skin of a treatment site during a radio-frequency invasive treatment, and facilitates storage, distribution, management, and assembly as a consumable by compact design.

## Description

### [Technical Field]

Embodiments of the present disclosure relate to a treatment tip for a handpiece, and more particularly, to a treatment tip for a scalp care handpiece that treats a scalp by penetrating microneedles into the scalp and applying electrical stimulation (radio frequency stimulation) and a handpiece including the same.

### [Background Art]

One of skin treatment methods spotlighted recently is a microneedling treatment. This is a method in which a needle with an extremely fine diameter is directly inserted into a skin to form treatment columns, a radio frequency (RF) is simultaneously applied to form fine scars for promoting regeneration of the skin, and the fine scars are healed to induce cell growth, thereby maximizing natural healing and regeneration of the skin.

An invasive radio frequency treatment device for the microneedling treatment generally includes a handpiece, a treatment tip, a controller, and the like. Microneedles serving as electrodes are installed at the treatment tip to be linearly movable. Accordingly, when the treatment tip is placed on a treatment site and the device is operated, the microneedles are simultaneously moved and inserted into a skin. In such a state, when a radio frequency signal is input, the microneedles electrically stimulate a corresponding skin layer.

The microneedling treatment can also be applied to a scalp. When the microneedling treatment is applied to the scalp, the dermal tissues of the scalp can be restructured and rearranged. In addition, the microneedling treatment can minimize the rupture of hair follicles and prevent scalp deformation or necrosis of cells. Moreover, the microneedling treatment can obtain the effect of stimulating the scalp's dermis to promote collagen synthesis and repair scalp damage and smoothing wrinkles.

On the other hand, heat is generated in a process of transferring or applying various types of stimulation to the skin during skin improvement and treatment using the invasive radio frequency treatment device. Since the generated heat may cause damage to the skin tissue in addition to burns on the skin surface, the heat needs be managed appropriately (to prevent burns). In addition, while the microneedling treatment is a treatment for minimizing damage to the skin and pain, a person to be treated inevitably feels pain due to the microneedle invasiveness. Accordingly, the invasive radio frequency treatment device employs a cooling technology for managing heat and pain caused during the treatment.

An example of the invasive radio frequency treatment device in the related art employing a cooling technology is disclosed in Korean Patent No. 10-2350161. Korean Patent No. 10-2350161 includes a cooling heat part disposed on a handpiece and a cooling plate part disposed on a treatment tip. The cooling heat part includes a heat exchange block and a cooling plate socket, and is mechanically coupled so that a cold heat transfer plate of the cooling plate part can transfer heat to the cooling plate socket.

However, when the mechanical coupling is implemented through the cold heat transfer plate, the size of the cold heat transfer plate increases, which not only increases the manufacturing cost but also causes inconveniences during storage, distribution, and assembly.

Patent Document 1: Korean Patent No. 10-2350161 (Publication Date: January 18, 2022)

### [Disclosure]

### [Technical Problem]

The present disclosure has been proposed in consideration of the aforementioned circumstances, and an object of the present disclosure is to provide a treatment tip for a scalp care handpiece that has a cooling function that can cool the skin of a treatment target site during radio-frequency invasive treatment to tighten skin tissue, relieve pain, and exhibit a skin soothing effect, and facilitates storage, distribution, management, and assembly with compact design, and a handpiece including the same.

### [Technical Solution]

A treatment tip for a scalp care handpiece according to an aspect of the present disclosure includes a cap part having a tip surface formed with a plurality of needle entry/exit holes, a cooling plate part mounted on the cap part, and a pair of terminal parts located on both sides of the cap part and accommodating both ends of the cooling plate part.

In the treatment tip according to an aspect of the present disclosure, a part of the cooling plate part is disposed on the tie surface so that an entire surface of the part is exposed to an outside, thereby directly contacting a skin during the treatment to cool a treatment site. Both ends of the cooling plate part are inserted into and fixed to the terminal parts, and are in close contact with ends of cooling plates to be described below within the terminal parts and receive cold heat when coupled to a handpiece body.

In the treatment tip according to an aspect of the present disclosure, the cooling plate part may preferably include a flat skin cooling plate disposed on the tip surface and formed with a plurality of needle penetration holes corresponding to the needle entry/exit holes, and a pair of cold heat transfer plates integrally formed to extend from both sides of the skin cooling plate.

The cold heat transfer plate may include a terminal region inserted into and fixed to the terminal part and an exposed region disposed between the terminal region and the skin cooling plate and exposed to an outside.

In the treatment tip according to an aspect of the present disclosure, the cap part may be provided with a seating guide that protrudes across both sides of the tip surface and the terminal part, prevents incorrect assembly of the cooling plate part, and guides the cooling plate part to be fixed at a designated position without shaking after assembly.

In the treatment tip according to an aspect of the present disclosure, a pair of snap joints may face each other at open end sides of the cap part opposite to the tip surface and may be formed at positions not overlapping with the pair of terminal parts.

A scalp care handpiece according to another aspect of the present disclosure includes a handpiece body including a cooling part and a treatment tip detachably provided to a tip of the handpiece body in a cartridge-like manner. The treatment tip is the treatment tip for a scalp care handpiece according to an aspect, the cooling part includes a pair of cooling plates, and the cooling plates are integrally configured with cooling terminals inserted into terminal parts of the treatment tip to come into contact with a cooling plate part.

In the scalp care handpiece according to another aspect of the present disclosure, the cooling part may include a heat exchange block configured to allow cooling water to flow in and the cooling water to be discharged to an outside via an inside of the heat exchange block, a pair of Peltier elements disposed in close contact with a first surface of the heat exchange block and a second surface opposite to the first surface, and the pair of cooling plates disposed in close contact with heat absorbing surfaces of the Peltier elements.

In the scalp care handpiece according to another aspect of the present disclosure, the heat absorbing surfaces of the pair of Peltier elements may be in close contact with the first surface and the second surface of the heat exchange block, and the cooling plate may be directly coupled to the heat exchange block with the Peltier element interposed between the cooling plate and the heat exchange block.

In the scalp care handpiece according to another aspect of the present disclosure, a heat exchange block with which the cooling plate makes heat exchangeable contact may be formed at the center thereof with a shaft through hole, a driving part that moves a needle part of the treatment tip up and down may be disposed behind the cooling part, and a driving shaft connecting the driving part and the needle part may be located in the shaft through hole, so that space efficiency can be improved.

In the scalp care handpiece according to another aspect of the present disclosure, a pair of release button parts may be provided in the handpiece body in correspondence to a pair of snap joints formed in the treatment tip.

In the scalp care handpiece according to another aspect of the present disclosure, the release button part may include a release button disposed so that a part of the release button is exposed to an outside through a button hole formed in a body case of the handpiece body, an elastic connector connected to one side of the release button and having an end fixed to a designated position inside the body case, a fastener formed on the other side of the release button at an opposite side of the elastic connector and coupled to the snap joint when the treatment tip is coupled, and an elastic support disposed inside the body case to elastically support a part of the release button so that the part protrudes outward from the body case through the button hole.

### [Advantageous Effects]

The scalp care handpiece according to the present disclosure can provide a function capable of effectively cooling the skin of a treatment site during a radio-frequency invasive treatment. In addition, through the configuration in which the treatment tip is provided with the terminal parts and the handpiece body-side terminals and the treatment tip-side terminals are connected within the terminal parts so that heat transfer is possible, energy loss can be prevented or minimized during the transfer of cooling energy to the treatment tip.

Moreover, unlike the configuration in the related art in which the terminal (cold heat transfer plate) extends by a predetermined length from the treatment tip for heat-transferrable contact with the handpiece body side, the present disclosure has compact design in which the terminal part is integrated into the treatment tip and the terminal part protruding to the outside is removed (deleted) from the treatment tip, thereby facilitating storage, distribution, management, and assembly of the treatment tip corresponding to a consumable.

### [Description of Drawings]

FIG. 1 is a perspective view of a scalp care handpiece according to an embodiment of the present disclosure.
FIG. 2 is an exploded perspective view of the present disclosure when a treatment tip is separated from a handpiece body in FIG. 1.
FIG. 3 is an assembled cross-sectional view when the scalp care handpiece according to an embodiment of the present disclosure is viewed from the direction of line A-A in FIG. 1.
FIG. 4 is an exploded perspective view of the treatment tip applied to the scalp care handpiece according to an embodiment of the present disclosure.
FIG. 5 is a cutaway perspective view when the treatment tip applied to the scalp care handpiece according to an embodiment of the present disclosure is viewed from the direction of line B-B in FIG. 2.
FIG. 6 is an enlarged view of a terminal part formed at a cap part of the treatment tip, wherein FIG. 6A is a perspective view of the terminal part and FIG. 6B is a cutaway perspective view of the terminal part.
FIG. 7 is a perspective view illustrating an internal configuration of a handpiece body applied to the scalp care handpiece according to an embodiment of the present disclosure.
FIG. 8 is a view illustrating main components extracted from FIG. 7.
FIG. 9 is an exploded perspective view of only a cooling part separately extracted from FIG. 8.
FIG. 10 is a cross-sectional view of main components of the present disclosure illustrating the coupling relationship between the treatment tip and the handpiece body (cross-sectional view when viewed from a direction orthogonal to line A-A in FIG. 1).

### [Mode for Invention]

Hereinafter, preferred embodiments of the present disclosure are described in detail.

Embodiments are provided to more fully explain the present disclosure to a person having ordinary knowledge in the art to which the present disclosure pertains. The following embodiments may be modified in various other forms, and the scope of the present disclosure is not limited to the following embodiments. Rather, these embodiments are provided to make the present disclosure more thorough and complete and to fully convey the spirit of the present disclosure.

Terms used in this specification are used to describe a specific embodiment, and are not intended to limit the present disclosure. In addition, in this specification, an expression of the singular number may include an expression of the plural number unless clearly defined otherwise in the context.

In this application, terms such as "includes", "comprises", or "has" are intended to designate the presence of features, numbers, steps, operations, components, parts, or combinations thereof of the present disclosure, and should be understood as not excluding in advance the possibility of the presence or addition of one or more other features, numbers, steps, operations, components, parts or combinations thereof.

In addition, the fact that a component is "in front of", "behind", "above", or "below" another component includes not only the case where it is directly adjacent to the other component and is disposed "in front of', "behind", "above", or "below" the other component, but also the case where another component is further disposed therebetween unless otherwise specified. In addition, the fact that a component is "connected" to another component includes not only the case where they are directly connected, but the case where they are indirectly connected unless otherwise specified.

The drawings are merely for enabling the spirit of the present disclosure to be understood, and it should not be interpreted that the scope of the present disclosure is limited by the drawings. In addition, in the drawings, a relative thickness or length or a relative size may be enlarged for convenience and the clarity of description.

A handpiece according to an embodiment of the present disclosure is a handpiece of a scalp care invasive radio-frequency treatment device that treats a scalp by penetrating microneedles into the scalp and applying electrical stimulation (radio frequency stimulation), and can relieve and improve hair loss or blackhead melanotrichia by inserting needles (microneedles) into the scalp of a patient and transmitting a radio-frequency (RF) through the inserted needles to improve the environment of the scalp.

Preferred embodiments of the present disclosure are described in detail with reference to the accompanying drawings.

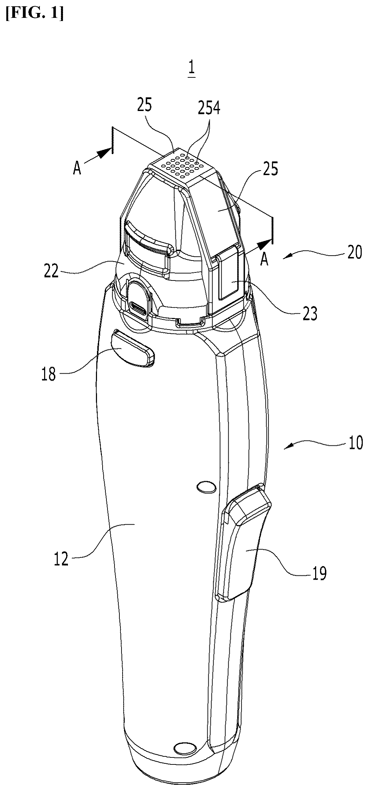

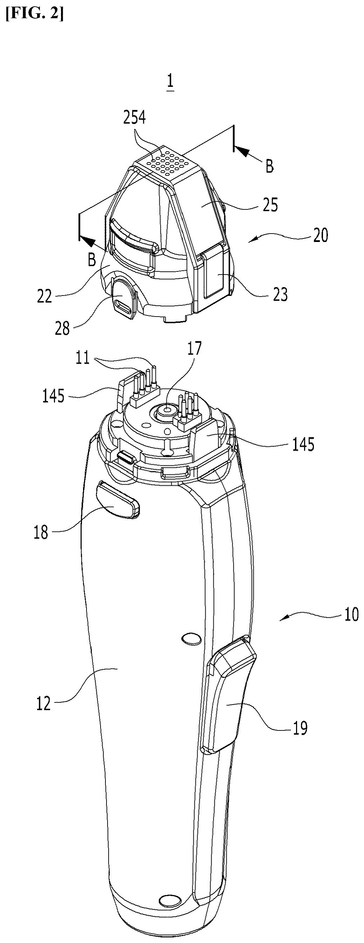

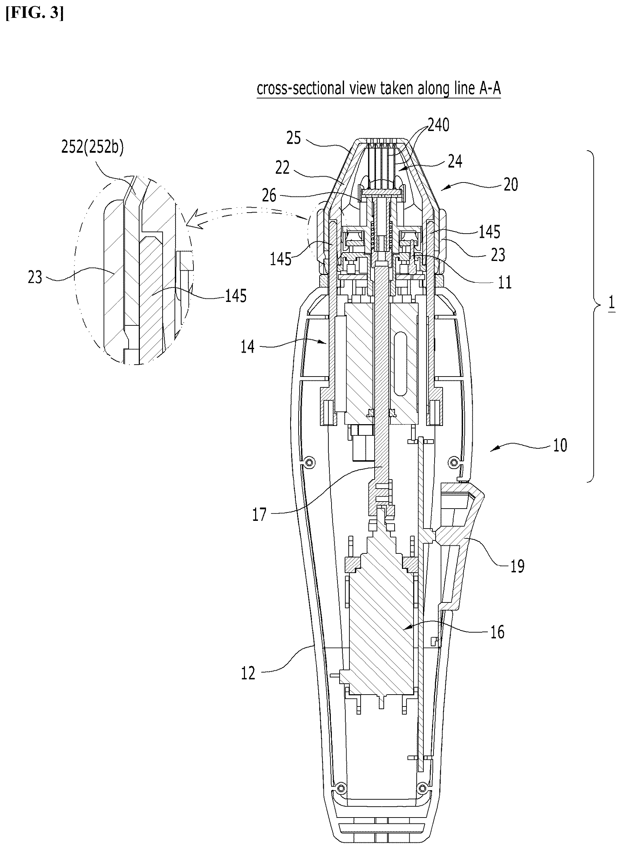

FIG. 1 is a perspective view of a scalp care handpiece according to an embodiment of the present disclosure, and FIG. 2 is an exploded perspective view of the present disclosure when a treatment tip is separated from a handpiece body. FIG. 3 is an assembled cross-sectional view when the scalp care handpiece according to an embodiment of the present disclosure is viewed from the direction of line A-A in FIG. 1. An overall configuration of the scalp care handpiece according to an embodiment of the present disclosure is described with reference to the drawings.

Referring to FIGS. 1 to 3, a scalp care handpiece 1 according to an embodiment of the present disclosure includes a handpiece body 10. The scalp care handpiece 1 according to an embodiment includes a treatment tip 20 mounted on a tip of the handpiece body 10. The treatment tip 20 is detachably mounted on the tip of the handpiece body 10 in a cartridge-like manner, and can be separated from the handpiece body 10 for easy replacement as needed.

The treatment tip 20 includes a plurality of needles 240 to which a radio-frequency (RF) current is applied from the handpiece body 10. The plurality of needles 240 can penetrate a target skin tissue of a person to be treated and transmit a radio frequency (RF). The treatment tip 20 can be provided in various types in which the number or arrangement form of needles 240 varies depending on the use purpose. Accordingly, an operator can mount the treatment tip 20 suitable for a treatment on the handpiece body 10, and perform a treatment.

The handpiece body 10 can be connected to a body (not illustrated) of a radio-frequency treatment device through a cable and exchange signals or information. The handpiece body 10 includes a driving part 16 that linearly drives a needle part 24 within the treatment tip 20. The handpiece body 10 may also include a radio-frequency generation part (not illustrated) that generates a radio frequency (RF) and applies the radio frequency (RF) to the needle part 24 including the needles 240.

The handpiece body 10 may include a control unit (not illustrated) and a cooling part 14. The control unit may include various control elements and a substrate on which the control elements are mounted. The control unit can be electrically connected to the driving part 16, the radio-frequency generation part, and the cooling part 14 and control them, and the cooling part 14 can generate cooling energy from cooling water supplied from the outside and a current applied under the control of the control unit, and transfer the cooling energy to the treatment tip 20.

The driving part 16 serves to reciprocate the needle part 24 including the plurality of needles 240 in a specific direction (up and down direction in the drawing) within a predetermined stroke range under the control of the control unit. The driving part 16 may be, for example, a motor. Of course, the driving part is not limited to a motor, and various types of actuators such as pneumatic actuators and solenoid-type actuators may be used as the driving part.

The radio-frequency generation part serves to convert power supplied from the body into a radio-frequency pulse under the control of the control unit. The radio-frequency generation part can convert the power supplied from the body into the radio-frequency pulse and transmit the radio-frequency pulse to the plurality of needles 240. Accordingly, the needles 240 can apply radio-frequency (RF) stimulation to the target skin tissue of the person to be treated in the state of penetrating the skin tissue.

The handpiece body 10 may be provided with a device operation button 19 that is exposed to the outside. The control unit can be electrically connected to the device operation button provided on the handpiece body 10. Accordingly, when a user operates the button on the handpiece body 10, the control unit recognizes the operation, generates a corresponding control signal, and transmits the control signal to the driving part 16, the radio-frequency generation part, and the cooling part 14, thereby controlling the operations of the driving part, the radio-frequency generation part, and the cooling part.

A driving shaft 17 can be connected to the driving part 16. A part of the driving shaft 17 can be exposed to the outside of the tip of the handpiece body 10. The driving shaft 17 exposed to the tip of the handpiece body 10 can be disposed inside the treatment tip 20 and coupled to a holder 26 mounted with the needle part 24. Thus, when the driving part 16 operates, the holder 26 coupled to the driving shaft 17 and the needle part 24 mounted thereon can move in a specific direction (up and down direction in the drawing).

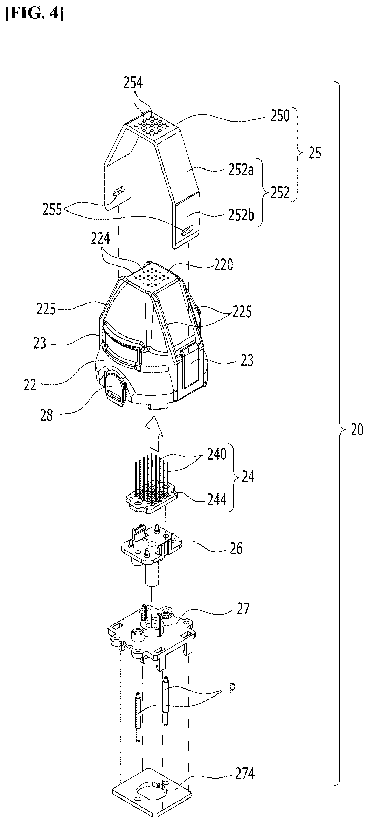

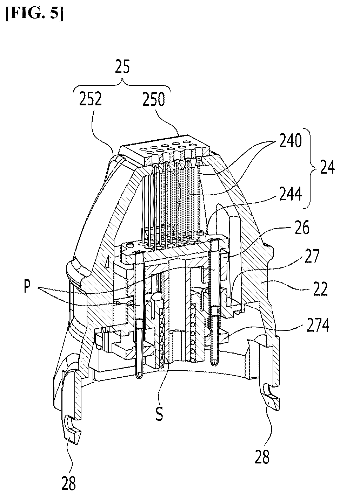

FIGS. 4 and 5 are views illustrating the treatment tip for the scalp care handpiece care according to an embodiment of the present disclosure. FIG. 4 is an exploded perspective view of the treatment tip and FIG. 5 is a cutaway perspective view when the treatment tip is viewed from the direction of line B-B in FIG. 2.

Referring to FIGS. 4 and 5, the treatment tip 20 includes the needle part 24 and a cap part 22. The needle part 24 includes the plurality of needles 240 that penetrate skin tissue and apply radio-frequency stimulation. The cap part 22 can define the outer shape of the treatment tip 20 and accommodate the needle part 24 therein. The cap part 22 may be made of an insulator such as plastic and detachably coupled to the tip of the handpiece body 10.

The needle part 24 of the treatment tip 20 includes the plurality of needles 240 and a substrate 244 on which the needles 240 are mounted. The substrate 244 can be mounted on the holder 26, and the holder 26 can be disposed inside the cap part 22 to be movable in a specific direction (up and down direction in the drawing). The specific directional movement of the holder 26 within the cap part 22 can be stably implemented without shaking by a lifting guide 27 installed on the inner lower portion of the cap part 22.

The holder 26 can be connected to the driving shaft 17 (see FIG. 3) protruding from the handpiece body 10. The needle part 24 including the needle 240 and the substrate 244 can be mounted on the holder 26. Thus, when the driving part operates, the holder 26 coupled to the driving shaft 17 and the needle part 24 mounted on the holder 26 can move relative to the cap part 22, which is a relative fixing body, in a specific direction (up and down direction in the drawing).

An intermediate substrate 274 that electrically connects the handpiece body 10 and the needle part 24 of the treatment tip 20 can be mounted on the lifting guide 27 fixedly disposed on the inner lower portion of the cap part 22. The intermediate substrate 274 may have a surface facing the needle part 24 and electrically connected to the substrate 244 of the needle part 24 via a connecting pin P, and an opposite surface electrically connected to radio-frequency supply terminals 11 (see FIG. 2) protruding from the tip of the handpiece body 10.

When the treatment tip 20 is separated from the handpiece body 10, the radio-frequency supply terminals 11 and the intermediate substrate 274 are physically separated, so that the electrical connection between the radio-frequency supply terminals 11 and the intermediate substrate 274 can be released. Conversely, when the treatment tip 20 is mounted on the handpiece body 10, the radio-frequency supply terminals 11 are electrically connected to the intermediate substrate 274, so that a radio-frequency current supplied from the handpiece body 10 can be stably supplied to the needle part 24 via the intermediate substrate 274.

The cap part 22 can be provided at the tip thereof with a tip surface 220. The tip surface 220 can be formed with a plurality of needle entry/exit holes 224 corresponding to the plurality of needles 240 in a one-to-one manner. The plurality of needles 240 can protrude to the outside by a predetermined length through the needle entry/exit holes 224 and penetrate skin tissue to transmit a radio frequency, or return to a treatment standby position inside the cap part 22, due to the movement of the needle part 24 relative to the cap part 22 in a specific direction.

The plurality of needles 240 serving as electrodes for transmitting radio frequency to a skin layer can be broadly divided into two groups. Needles belonging to the same group may have the same polarity, while needles belonging to different groups may have opposite polarities. For example, when the needles belonging to one of the two groups are positive (+) electrodes, the needles belonging to the other group may form negative (-) electrodes.

In the treatment standby position, the plurality of needles 240 are located inside the cap part 22 and are therefore not exposed to the outside. When the operator operates the device and issues a treatment start command in such a state, the driving part 16 operates in a specific direction, so that the plurality of needles 240 are simultaneously withdrawn to the outside by a predetermined length through the needle entry/exit holes 224. Consequently, the needles 240 can penetrate the skin to be treated and apply radio-frequency stimulation.

The treatment tip 20 may include a pair of terminal parts 23. The pair of terminal parts 23 can be formed to define a predetermined space (hereinafter, referred to as a "heat transfer path") on one side of the cap part 22 and the other side opposite to the one side. The pair of terminal parts 23 defining the heat transfer path can be integrally formed with the cap part 22. For example, the pair of terminal parts 23 can be formed as a single object (integrated with the cap part) on one side of the cap part 22 and the other side opposite to the one side through injection molding.

A cooling plate part 25 can be mounted on the cap part 22 of the treatment tip 20. The cooling plate part 25 serves to receive cooling energy from the handpiece body 10 and cool the skin to be treated. A part of the cooling plate part 25 is disposed on the tip surface 220 and exposed to the outside and the other part of the cooling plate part 25 is inserted into and fixed to the terminal part 23. Thus, when the cooling plate part 25 is coupled to the handpiece body 10, the cooling plate part 25 can be in close contact with a part of the cooling part 14 to be described below within the terminal part 23 and receive cold heat.

The portion of the cooling plate part 25 that is disposed on the tip surface 220 and exposed to the outside directly contacts the skin to be treated during a radio-frequency treatment, thereby cooling a treatment site. This can prevent burns caused by radio-frequency heat, tighten the skin tissue at the treatment site, and relieve pain during the treatment. In addition, it can also have a soothing effect on the skin at the treatment site.

The cooling plate part 25 may include a skin cooling plate 250 and a cold heat transfer plate 252. The skin cooling plate 250 is a portion that is disposed on the tip surface 220 and directly contacts the skin during the radio-frequency treatment. The cold heat transfer plates 252 can be configured as a pair extending integrally from both sides of the skin cooling plate 250. The cold heat transfer plate 252 serves as an intermediate medium that receives cooling energy from the cooling part 14 and provides the cooling energy to the skin cooling plate 250.

The skin cooling plate 250 can be configured as a flat plate with an area corresponding to the area of the tip surface 220 of the cap part 22. The skin cooling plate 250 can be formed with a plurality of needle penetration holes 254 through which the plurality of needles 240 pass. Each of the needle penetration holes 254 can be arranged to match the needle entry/exit hole 224 formed in the tip surface 220. In addition, the diameter of the needle penetration hole 254 may be formed to be the same as the diameter of the needle entry/exit hole 224.

The cold heat transfer plate 252 can be divided into a terminal region 252b and an exposed region 252a. The terminal region 252b is a portion that is inserted into and fixed to the aforementioned terminal part 23 integrally configured on both sides of the cap part 22, and the exposed region 252a refers to a portion that is located between the terminal region 252b and the skin cooling plate 250 and is exposed to the outside. The terminal region 252b and the exposed region 252a can be formed to have the same thickness and width in consideration of heat conduction characteristics.

A seating guide 225 can be formed on the cap part 22 of the treatment tip 20. The seating guide 225 can be formed as a pair of rail structures that protrude across both sides of the tip surface 220 and the terminal part 23, thereby forming a space where the cold heat transfer plate 252 can be stably seated and fixed. The seating guide 225 serves to prevent incorrect assembly during mounting of the cooling plate part 25 and allow the cooling plate part 25 to be fixed at a designated position without shaking after the mounting.

The cooling plate part 25 may be made of a metal material with high thermal conductivity and corrosion resistance in consideration of heat transfer efficiency, cooling efficiency due to skin contact, corrosion resistance, and the like. As a preferred example, the cooling plate part 25 may be made of aluminum. Of course, the present disclosure is not limited thereto.

In FIGS. 4 and 5, reference numeral 28 denotes a snap joint provided on the cap part 22 for stable coupling with the handpiece body 10. The snap joint 28 can be configured as a pair integrally formed with the cap part 22 at open end sides of the cap part 22 located opposite to the tip surface 220. Preferably, as illustrated in the drawing, the snap joints can face each other at the open end sides and be disposed at positions not overlapping with the pair of terminal parts 23.

In FIG. 5, reference numeral S denotes a spring interposed between the holder 26 and the lifting guide 27 to generate a restoring force for returning the holder 26 to the treatment standby position.

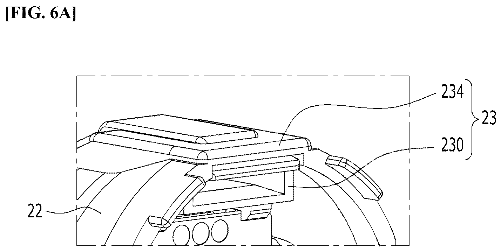

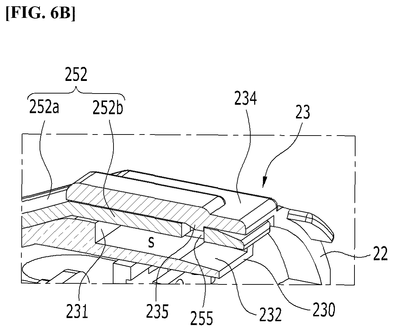

FIG. 6 is an enlarged view of the terminal part formed at the cap part of the treatment tip, wherein FIG. 6A is a perspective view of the terminal part and FIG. 6B is a cutaway perspective view of the terminal part.

Referring to FIG. 6, the terminal part 23 provided on the treatment tip 20 provides a space for precise and close contact between the cooling plate part 25 and a part (cooling terminals 145) to be described below of the cooling part 14. The terminal part 23 may include a trench-shaped lower receiver 230 recessed inward of the cap part 22 and an upper cover 234 covering the lower receiver 230, and have a three-dimensional structure in which one side facing the tip surface 220 and the other side facing the handpiece body 10 are opened.

When the treatment tip 20 is assembled, one side of the terminal region 252b constituting the cold heat transfer plate 252 can be tightly fixed to an inner surface of the upper cover 234. In the state in which the terminal region 252b is inserted into and fixed to the terminal part 23, a connection space s with a sufficient volume can be formed between the terminal region 252b and a bottom of the lower receiver 230 to allow the part (cooling terminals 145) of the cooling part 14 to be in tight contact with the terminal region 252b.

A wedge-shaped protrusion 235 and a hole 255 can be formed on the inner surface of the upper cover 234 and the terminal region 252b, respectively, for firm coupling. In addition, a stepped portion 231 can be formed at one open end of the lower receiver 230 to allow the part (cooling terminals 145) of the cooling part 14 to be accurately stopped at an intended position. In addition, a slope guide surface 232 can be formed on the surface of the bottom of the lower receiver 230 facing the upper cover 234.

The slope guide surface 232 serves to guide the part (cooling terminals 145, see FIG. 2) of the cooling part 14 to gradually move toward terminal region 252b to be stably close contact with the terminal region 252b during the insertion process of the part of the cooling part 14 into the terminal part 23. As illustrated in FIG. 6B, the slope guide surface 232 can be formed to be inclined upward from the other open end of the lower receiver 230, into which the part (cooling terminals 145) of the cooling part 14 is inserted, toward the inside of the lower receiver 230.

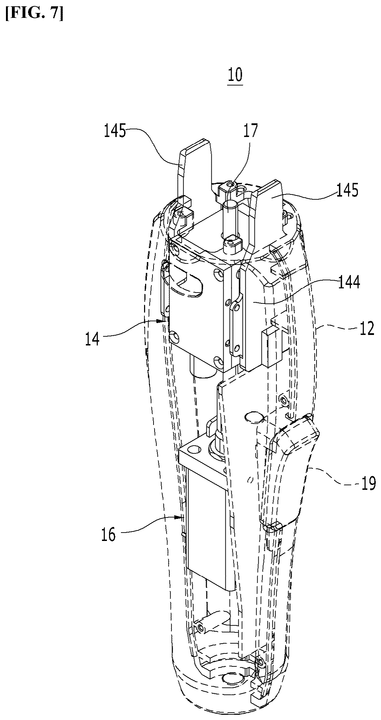

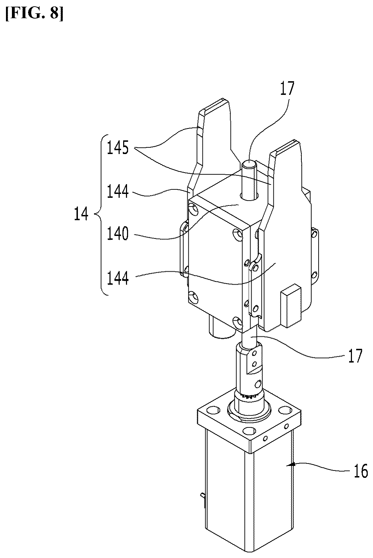

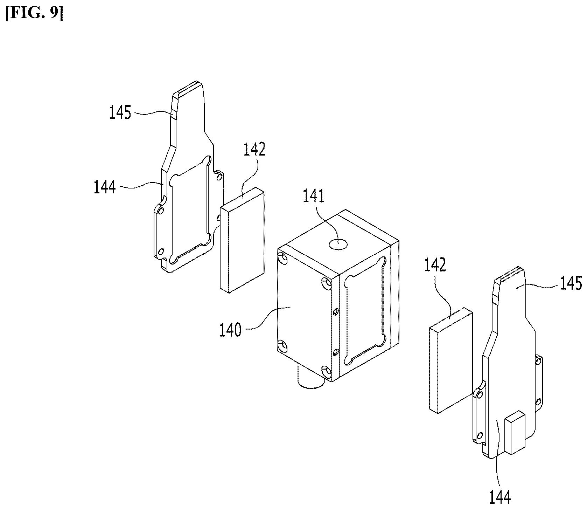

FIGS. 7 to 9 are views illustrating the handpiece body of the scalp care handpiece according to an embodiment of the present disclosure. FIG. 7 is a perspective view illustrating an internal configuration of the handpiece body, and FIG. 8 is a view illustrating main components extracted from FIG. 7. FIG. 9 is an exploded perspective view of only the cooling part separately extracted from FIG. 8.

Referring to FIGS. 7 to 9 together with FIG. 3, the handpiece body 10 includes a body case 12 and the cooling part 14 disposed therein. The cooling part 14 includes cooling plates 144, and the cooling terminals 145 can be integrally formed with the cooling plate 144. When the treatment tip 20 is coupled to the handpiece body 10, the cooling terminals 145 can be inserted into the terminal parts 23 of the treatment tip 20 and brought into close contact with the terminal region 252b of the aforementioned cold heat transfer plate 252 to enable heat transfer with the terminal region 252b.

The cooling part 14 includes a heat exchange block 140, a pair of Peltier elements 142, and the pair of cooling plates 144. A cooling water line through which cooling water flows in and is discharged to the outside via the inside of the heat exchange block 140 can be formed in the heat exchange block 140. The pair of Peltier elements 142 can be disposed in close contact with a first surface of the heat exchange block 140 and a second surface opposite to the first surface. In addition, the cooling plate 144 can be disposed in close contact with each Peltier element 142.

When a current is applied to the Peltier element 142, the Peltier element 142 absorbs heat on one side to cause cooling and generates heat on the opposite side according to the Peltier effect. In an embodiment, the cooling plate 144 is in close contact with a heat absorbing surface (surface that absorbs heat) of each Peltier element 142, and a heat generating surface (surface that generates heat) of each Peltier element 142 located opposite to the heat absorbing surface can be disposed in close contact with the first and second surfaces of the heat exchange block 140.

With such a configuration, when a current is applied to the Peltier element 142 in response to a device operation command, the cooling plate 144 in close contact with the heat absorbing surface of each Peltier element 142 can be cooled by a cooling effect occurring on the heat absorbing surface. In addition, since the heat generated from the heat generating surface of each Peltier element 142 is discharged to the outside through the cooling water circulating within the heat exchange block 140 through heat exchange with the heat exchange block 140, the cooling plate 144 can continuously maintain a cooled state.

The cooling terminal 145 can be integrally formed with the cooling plate 144, and can extend by a predetermined length toward the treatment tip 20. Accordingly, when the treatment tip 20 is coupled to the handpiece body 10, the cooling terminal 145 can be inserted into the terminal part 23 of the treatment tip 20 and make close contact with the cold heat transfer plate 252 within the terminal part 23 to enable heat transfer (see the partially enlarged view in FIG. 3).

This allows cooling energy generated by the Peltier element 142 to be finally transferred to the skin cooling plate 250 via the cold heat transfer plate 252 of the treatment tip 20, so that a treatment site during a radio-frequency treatment can be effectively cooled. As a result, burns caused by radio-frequency heat generated during the treatment can be prevented and pain can be relieved during the treatment. In addition, a skin at the treatment site is soothed by the cooling effect.

The cooling plate 144 and the heat exchange block 140 may each be made of a metal material with high thermal conductivity. Since cooling water circulates in the heat exchange block 140, a metal material with corrosion resistance is preferred. As a preferred example, the cooling plate 144 may be made of copper or aluminum. In addition, the heat exchange block 140 may be made of aluminum with high thermal conductivity and corrosion resistance. Of course, the present disclosure is not limited thereto.

The cooling plate 144 can be directly coupled to the heat exchange block 140 with the Peltier element 142 interposed between the cooling plate 144 and the heat exchange block 140. For example, the cooling plate 144 can be fastened to the heat exchange block 140 with the Peltier element 142 interposed between the cooling plate 144 and the heat exchange block 140 by a fastening member such as a bolt and stably fixed thereto. In such a case, the fastening member preferably uses an engineering plastic fastening member to ensure sufficient fastening strength between the cooling plate 144 and the heat exchange block 140 while preventing heat loss.

In the scalp care handpiece 1 according to an embodiment, the heat exchange block 140, with which the cooling plate 144 makes heat exchangeable contact, can be formed in a hollow structure in which a shaft through hole 141 is formed in the center of the heat exchange block 140. In addition, the driving part 16 that moves the needle part 24 of the treatment tip 20 up and down can be disposed behind the heat exchange block 140. In such a case, the driving shaft 17 connecting the driving part 16 and the needle part 24 is located in the shaft through hole 141, thereby eliminating the effort for separately securing an arrangement space for the driving shaft 17.

The body case 12 may have a configuration in which a cap member (not illustrated) is coupled to a tip (upper portion in the drawing) of a unit case (not illustrated) that is divided into two pieces and coupled together. In such a case, the cap member may have a hole (not illustrated) through which the driving shaft 17 and the plurality of radio-frequency supply terminals 11 pass. A radio-frequency output substrate (not illustrated) that transmits a radio-frequency current to the intermediate substrate 274 described above through the radio-frequency supply terminals 11 can be disposed.

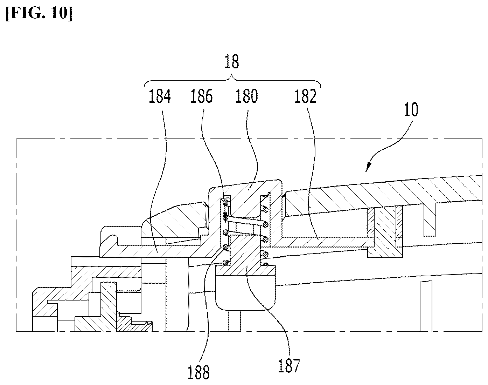

FIG. 10 is a cross-sectional view of main components of the present disclosure illustrating the coupling relationship between the treatment tip and the handpiece body (cross-sectional view when viewed from a direction orthogonal to line A-A in FIG. 1).

Referring to FIG. 10, the handpiece body 10 can be provided with a pair of release button parts 18 for maintaining a coupled state by fixing the treatment tip 20, or releasing the coupling so that the treatment tip 20 can be separated from the handpiece body 10. The release button parts 18 are configured to correspond to the pair of snap joints 28 formed on the treatment tip 20 and may each include a release button 180, an elastic connector 182, a fastener 184, and an elastic support 186.

The release button 180 can be disposed so that a part of the release button is exposed to the outside through a button hole (not illustrated) formed in the body case 12 of the handpiece body 10. The elastic connector 182 can be connected to one side of the release button 180, and its end can be fixed to a designated position inside the body case 12. In addition, the fastener 184 can be formed on the other side of the release button 180 at an opposite side of the elastic connector 182, and can be engaged with the snap joint 28 when the treatment tip 20 is coupled.

At an end of each of the snap joint 28 and the fastener 184, hooks or ring-shaped fastening pieces (not illustrated) protruding toward each other can be formed so that the fastening pieces can be engaged with each other when the treatment tip 20 is coupled. In addition, the elastic support 186 is disposed inside the body case 12 to provide elasticity so that a part of the release button 180 protrudes outward from the body case 12 through the button hole.

As a preferred example, as illustrated in the drawing (FIG. 10), the elastic support 186 may include a support 187 located further inside the body case 12 than the release button 180, and a spring 188 installed between the support 187 and the release button 180 to apply elasticity to push the release button 180 outward from the body case 12. Of course, the present disclosure is not limited thereto, and the same function can also be implemented using various elastic components or support structures.

The effects of the scalp care handpiece according to an embodiment of the present disclosure described above are described below in conjunction with the operation of the handpiece.

Referring to the accompanying drawings, in the handpiece 1 according to an embodiment, the treatment tip 20 is detachably mounted at the tip of the handpiece body 10 in a cartridge-like manner. The treatment tip 20 is provided in various types in which the number or arrangement form of needles varies depending on the use purpose. Accordingly, an operator can couple the treatment tip 20 suitable for a treatment to the handpiece body 10, and perform a treatment.

A radio-frequency treatment using the handpiece 1 according to an embodiment can be performed by operating a device operation button in a state in which the tip of the treatment tip 20 is in contact with a treatment site (skin to be treated). When the button of the handpiece body 10 is operated, the control unit recognizes the operation, generates a corresponding control signal, and transmits the control signal to the driving part 16, the radio-frequency generation part, and the cooling part 14, thereby implementing the operations of the device for the radio-frequency treatment.

The driving part 16 operates to withdraw the needle part 24 located on the treatment tip 20 upon receiving a treatment start command from the control unit, and the radio-frequency generation part converts power supplied from the body into a radio-frequency pulse and transmits the radio-frequency pulse to the needles of the needle part 24. In addition, the cooling part 14 generates cooling energy by operating the Peltier element 142 by a current applied under the control of the control unit.

When the driving part 16 is operated by the control unit, the plurality of needles 240 of the needle part 24 are withdrawn outward from the tip of the treatment tip 20 by a predetermined length and penetrate the skin tissue of the treatment site. In such a state, the plurality of needles 240 receive a radio-frequency current from the radio-frequency generation part and apply radio-frequency (RF) stimulation to the corresponding skin manipulation, thereby achieving effects such as improved skin elasticity and skin regeneration.

When the treatment tip 20 is coupled to the handpiece body 10, the pair of cooling terminals 145 extending from the tip of the handpiece body 10 toward the treatment tip 20 are inserted into the terminal parts 23 of the treatment tip 20 and are in close contact with the cooling plate part 25 constituting the treatment tip 20 within the terminal parts 23, thereby enabling heat transfer. Accordingly, cooling energy generated by the cooling part 14 of the handpiece body 10 can be effectively transferred to the cooling plate part 25.

A portion (skin cooling plate) of the cooling plate part 25, which is disposed at the tip of the treatment tip 20 and exposed to the outside, directly contacts a skin to be treated during a radio-frequency treatment, thereby cooling a treatment site. This can prevent burns caused by radio-frequency heat during the treatment, tighten a skin tissue at the treatment site, and reduce pain during the treatment. In addition, it can also have a soothing effect on the skin at the treatment site.

As described above, the scalp care handpiece according to an embodiment can provide a function capable of effectively cooling a skin at a treatment site during a radio-frequency invasive treatment. In addition, through the configuration in which the treatment tip is provided with the terminal parts and the handpiece body-side terminals and the treatment tip-side terminals are connected within the terminal parts so that heat transfer is possible, energy loss can be prevented or minimized during the transfer of cooling energy to the treatment tip.

Moreover, unlike the configuration in the related art in which the terminal (cold heat transfer plate) extends by a predetermined length from the treatment tip for heat-transferrable contact with the handpiece body side, the present disclosure has compact design in which the terminal part is integrated into the treatment tip and the terminal part protruding to the outside is removed (deleted) from the treatment tip, thereby facilitating storage, distribution, management, and assembly of the treatment tip corresponding to a consumable.

The above description is merely a description of the technical spirit of the present disclosure, and those skilled in the art may change and modify the present disclosure in various ways without departing from the essential characteristic of the present disclosure.

Accordingly, the embodiments disclosed in the present disclosure should not be construed as limiting the technical spirit of the present disclosure, but should be construed as describing the technical spirit of the present disclosure. The technical spirit of the present disclosure is not restricted by the embodiments. The range of protection of the present disclosure should be construed based on the following claims, and all of technical spirits within an equivalent range of the present disclosure should be construed as being included in the scope of rights of the present disclosure.

## Claims

1. A treatment tip for a scalp care handpiece, comprising:
a cap part having a tip surface formed with a plurality of needle entry/exit holes;
a cooling plate part mounted on the cap part; and
a pair of terminal parts located on both sides of the cap part and accommodating both ends of the cooling plate part.

2. The treatment tip for a scalp care handpiece of claim 1, wherein a part of the cooling plate part is disposed on the tip surface so that an entire surface of the part is exposed to an outside, and
both ends of the cooling plate part are inserted into and fixed to the terminal parts.

3. The treatment tip for a scalp care handpiece of claim 1, wherein the cooling plate part comprises:
a flat skin cooling plate disposed on the tip surface and formed with a plurality of needle penetration holes corresponding to the needle entry/exit holes; and
a pair of cold heat transfer plates formed to extend from both sides of the skin cooling plate,
wherein the cold heat transfer plate includes a terminal region inserted into and fixed to the terminal part and an exposed region disposed between the terminal region and the skin cooling plate and exposed to an outside.

4. The treatment tip for a scalp care handpiece of claim 1, wherein the cap part is provided with a seating guide that protrudes across both sides of the tip surface and the terminal part.

5. The treatment tip for a scalp care handpiece of claim 1, wherein a pair of snap joints face each other at open end sides of the cap part opposite to the tip surface and are formed at positions not overlapping with the pair of terminal parts.

6. A scalp care handpiece comprising:
a handpiece body including a cooling part; and
a treatment tip detachably provided to a tip of the handpiece body in a cartridge-like manner,
wherein the treatment tip is the treatment tip according to any one of claims 1 to 5,
the cooling part includes a pair of cooling plates, and
the cooling plates are integrally configured with cooling terminals inserted into terminal parts of the treatment tip to come into contact with a cooling plate part.

7. The scalp care handpiece of claim 6, wherein the cooling part comprises:
a heat exchange block configured to allow cooling water to flow in and the cooling water to be discharged to an outside via an inside of the heat exchange block;
a pair of Peltier elements disposed in close contact with a first surface of the heat exchange block and a second surface opposite to the first surface; and
the pair of cooling plates disposed in close contact with heat absorbing surfaces of the Peltier elements.

8. The scalp care handpiece of claim 7, wherein the heat absorbing surface of each Peltier element is in close contact with the first surface and the second surface of the heat exchange block, and
the cooling plate is directly coupled to the heat exchange block with the Peltier element interposed between the cooling plate and the heat exchange block.

9. The scalp care handpiece of claim 6, wherein the cooling part further comprises:
a heat exchange block with which the cooling plate makes heat exchangeable contact,
wherein the heat exchange block is formed at a center thereof with a shaft through hole,
a driving part that moves a needle part of the treatment tip up and down is disposed behind the cooling part, and
a driving shaft connecting the driving part and the needle part is located in the shaft through hole.

10. The scalp care handpiece of claim 6, wherein a pair of release button parts are provided in the handpiece body in correspondence to a pair of snap joints formed in the treatment tip.

11. The scalp care handpiece of claim 9, wherein the release button part comprises:
a release button disposed so that a part of the release button is exposed to an outside through a button hole formed in a body case of the handpiece body;
an elastic connector connected to one side of the release button and having an end fixed to a designated position inside the body case;
a fastener formed on the other side of the release button at an opposite side of the elastic connector and coupled to the snap joint when the treatment tip is coupled; and
an elastic support disposed inside the body case to elastically support a part of the release button so that the part protrudes outward from the body case through the button hole.
